# FASCICULE DE BREVET EUROPEEN

(11) **EP 0 604 300 B1**
(45) Date de publication et mention de la délivrance du brevet: **15.03.2000**
(21) Numéro de dépôt: 93403108.9
(22) Date de dépôt: 20.12.1993
(51) Int. Cl.: G01F 11/02, A61M 5/315

(54) **Pompe volumétrique réglable telle que seringue, pistolet, pipette**
Veränderliche volumetrische Pumpe, etwa eine Spritze, Spritzpistole oder Pipette
Adjustable volumetric pump such as a syringe, gun or pipette

(30) Priorité: 21.12.1992 FR 9215338
(43) Date de publication de la demande: 29.06.1994
(73) Titulaire: SOCIETE DE FABRICATIONS, BARBOT GENIA S.A., F-44120 Bertou (FR)
(72) Inventeur: Comminges, Bernard ,, F-44200 Nantes (FR)
(74) Mandataire: Dawidowicz, Armand

(56) Documents cités:
- CH-A- 449 180
- DE-C- 945 048
- FR-A- 2 628 011
- GB-A- 429 729
- GB-A- 524 916

## Description

La présente invention concerne une pompe volumétrique réglable telle que seringue, pistolet, pipette notamment à usage thérapeutique, médical, vétérinaire, analytique ou autre.

Les pompes volumétriques réglables, en particulier, les seringues automatiques, du type comportant un corps cylindrique constituant le réservoir de la pompe fermé par un fond, un piston monté coulissant axialement à l'intérieur dudit corps pour injecter une quantité variable de liquide contenue entre la tête du piston et ledit fond du corps cylindrique, et un support de corps de réservoir maintenu fixe à une extrémité dudit corps de réservoir, sont connues.

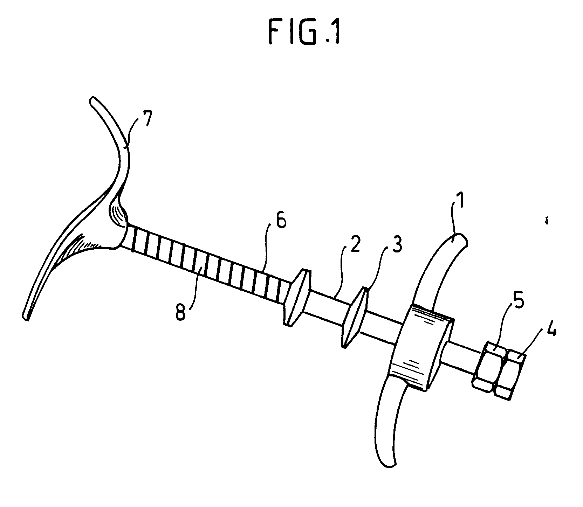

Une telle seringue connue, représentée à la figure 1 sans son corps de seringue formant réservoir et sans l'embout, comporte un piston 8 animé d'un mouvement alternatif à l'intérieur du corps formant réservoir, ledit piston étant actionné au moyen d'un organe de poussée encore appelé béquille 7. Entre le piston 8 et les parois du logement longitudinal ou axial à l'intérieur duquel se déplace axialement le piston 8 dans le réservoir est disposée une vis de réglage 2 filetée qui coopère avec le taraudage du chapeau 1 constituant le support du corps de réservoir de manière à pénétrer plus ou moins à l'intérieur dudit corps de réservoir. L'autre extrémité de cette vis de réglage qui pénètre à l'intérieur dudit corps comprend un épaulement 5 sur lequel vient en butée la tête de piston 4 du piston 8 quand ce dernier est en déplacement axial vers l'extérieur du corps de réservoir. Un contre-écrou 3 est disposé sur la vis de réglage et permet d'immobiliser ladite vis en une position appropriée. Le réglage d'une dose d'injection particulière se fait donc par rotation de la vis dans un sens ou dans l'autre de manière à pénétrer plus profondément dans le réservoir ou au contraire à sortir plus du réservoir de manière à s'éloigner du fond du réservoir, de telle sorte que la course du piston soit plus ou moins importante. Lorsque la position requise a été déterminée, cette position est fixée au moyen du contre-écrou 3 qui vient se visser à l'intérieur du chapeau 1.

L'inconvénient d'un tel dispositif réside dans ses limites d'utilisation. En effet, en raison du dispositif de réglage, il n'est pas possible d'avoir des variations de volume d'injection importantes sans entraîner un encombrement rendant l'ensemble difficilement manipulable. En effet, lorsque le volume d'injection est important, la vis de réglage 2 est sortie au maximum du corps de réservoir et la béquille 7 se trouve alors très éloignée du chapeau 1 du corps de réservoir, ce qui ne facilite pas la préhension du dispositif. A l'inverse, lorsque la dose d'injection est faible, la béquille 7 se trouve assez proche du chapeau 1. Du fait de cette variation de la distance entre le chapeau 1 et la béquille 7, les variations de volumes sont limitées et doivent être telles que, dans les positions extrêmes, une manipulation du dispositif reste efficace.

Le brevet DE-C-945.048 décrit une pompe du type comprenant une bouteille traversée par un logement axial comportant un piston, ledit logement axial constituant un réservoir fermé à l'une de ses extrémités par un élément formant bouchon venant se visser sur l'extrémité dudit réservoir de manière à permettre un réglage de la quantité de volume injecté. En conséquence, seul ce bouchon permet le réglage du volume d'injection. Du fait que ce bouchon ne comporte pas de moyen d'immobilisation, il peut en résulter une imprécision et un dérèglement de l'ensemble au moment de l'injection. En outre, il n'est pas possible avec un tel dispositif de couvrir une grande plage d'injection du fait que le corps du réservoir ne peut être modifié.

Le document CH-A-449.180 décrit une seringue à répétition comprenant au moins un cylindre dans lequel coulisse un piston. Un mécanisme commande l'avance du piston pas à pas dans le cylindre. Chaque pas correspond à une dose du produit à injecter même si le réglage de la quantité de produit à injecter est fonction du pas défini pour l'avance du piston.

Le document GB-A-524.916 décrit à nouveau une seringue constituée d'un corps de réservoir, d'un support et d'un piston, le réglage de la quantité de produit à injecter s'effectuant par réglage de la position de recul du piston par rapport au support du corps du réservoir.

Le brevet GB-A-429.729 décrit quant à lui une pompe volumétrique réglable dans laquelle le réservoir est formé de deux tubes A et B télescopiques animés d'un mouvement relatif axial l'un par rapport à l'autre et susceptibles d'être immobilisés dans une position réglable prédéterminée de telle sorte que le coulissement de l'un des tubes dans l'eau détermine la quantité de produit à injecter. Ce dispositif ne comprend pas de piston monté coulissant axialement à l'intérieur du corps de réservoir de manière à injecter une quantité variable de liquide contenue entre la tête du piston et le fond du réservoir. Ce brevet présente les mêmes inconvénients, à savoir un encombrement important du dispositif lorsque les doses de quantité à injecter sont importantes, que les documents précités.

Le but de la présente invention est de proposer une pompe volumétrique réglable dont la distance entre l'organe de poussée du piston et le support du corps de seringue est constante indépendamment de la quantité de volume injectée de manière à faciliter la manipulation de l'ensemble.

L'invention concerne à cet effet une pompe volumétrique réglable telle que seringue, pistolet, pipette, du type comportant un corps, de préférence cylindrique, et constituant le réservoir de la pompe fermé par un fond, un piston monté coulissant axialement à l'intérieur dudit corps pour injecter une quantité variable de liquide contenue entre la tête du piston et ledit fond du corps, et un support du corps de réservoir, caractérisée en ce que le réservoir est animé d'un mouvement axial à l'intérieur du support du corps de réservoir et est susceptible d'être immobilisé dans ledit support en au moins une position appropriée correspondant à un volume d'injection choisi, ledit piston étant fixe par rapport audit support en position de repos de la pompe. Selon une forme de réalisation préférée de l'invention, le piston est actionné par un organe qui le limite dans son mouvement axial vers l'extérieur du réservoir et le ramène toujours à une même position limite de manière à ce que le support du corps de réservoir soit maintenu, indépendamment du volume d'injection, à une distance déterminée toujours identique de la tête du piston dans la position de repos correspondant à une position de recul maximal dans laquelle le piston ne subit aucune force d'enfoncement axial à l'intérieur du réservoir.

Le déplacement axial du corps de réservoir dans le support de réservoir est réalisé au moyen d'un filetage disposé sur la face externe du corps de réservoir qui coopère avec un taraudage du support du corps de réservoir.

L'organe d'immobilisation du corps de réservoir dans une position appropriée à l'intérieur du support de réservoir peut être constitué par un contre-écrou vissé sur le corps dudit réservoir et venant en butée au cours du vissage contre un épaulement radial interne dudit support de réservoir.

D'autres caractéristiques et avantages de l'invention apparaîtront encore à la lecture de la description qui suit et des dessins joints, lesquels description et dessins sont donnés surtout à titre d'exemple. Dans ces dessins :
la figure 1 représente une vue de côté d'une seringue à volume d'injection réglable correspondant à l'état de la technique ;
la figure 2 représente une vue de côté en coupe d'une pompe volumétrique réglable, en l'occurrence une seringue automatique à alimentation par tuyau, conforme à l'invention ;
la figure 3 représente une vue de côté d'une pompe volumétrique réglable constituée de nouveau par une seringue automatique alimentée au moyen d'un flacon ;
les figures 4 et 5 représentent deux vues en coupe de réservoirs correspondant à des volumes respectifs de 2 et 5 ml et
la figure 6 représente une vue de profil d'un réservoir conforme à l'invention.

La pompe volumétrique réglable qui va être décrite ci-dessous correspond à une seringue. Cependant, toutes les caractéristiques de construction qui seront décrites ci-après pourraient de la même façon s'appliquer à un pistolet ou à une pipette.

Cette seringue comporte un corps 9, de préférence cylindrique, constituant le réservoir de ladite seringue, ce corps étant fermé par un fond (13, 14). Cette seringue comprend en outre un piston 19 monté coulissant axialement à l'intérieur dudit corps 9 de manière à injecter une quantité variable de liquide contenue entre la tête du piston 18 et ledit fond 13 du corps.

Enfin, cette seringue comprend en outre un support 27 du corps de réservoir 9.

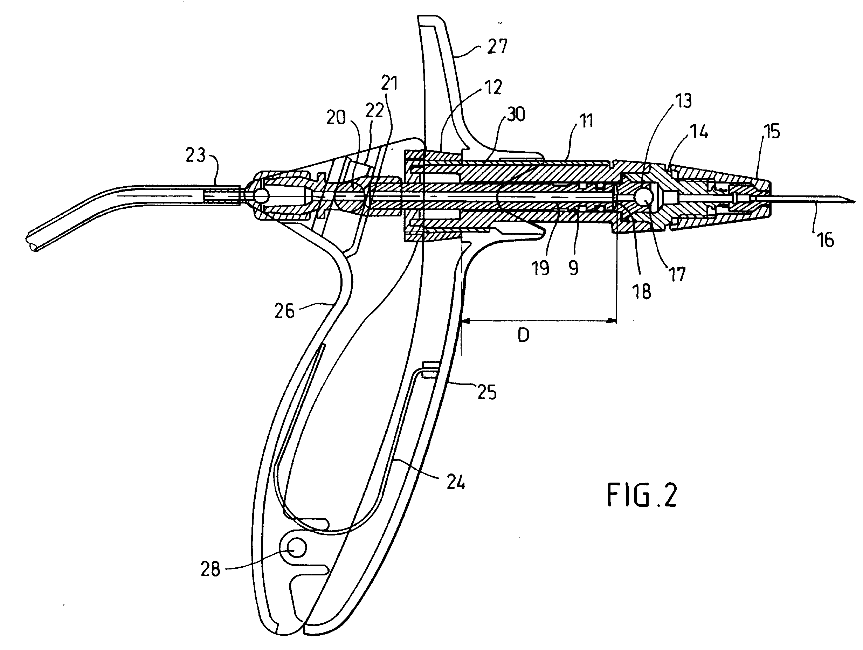

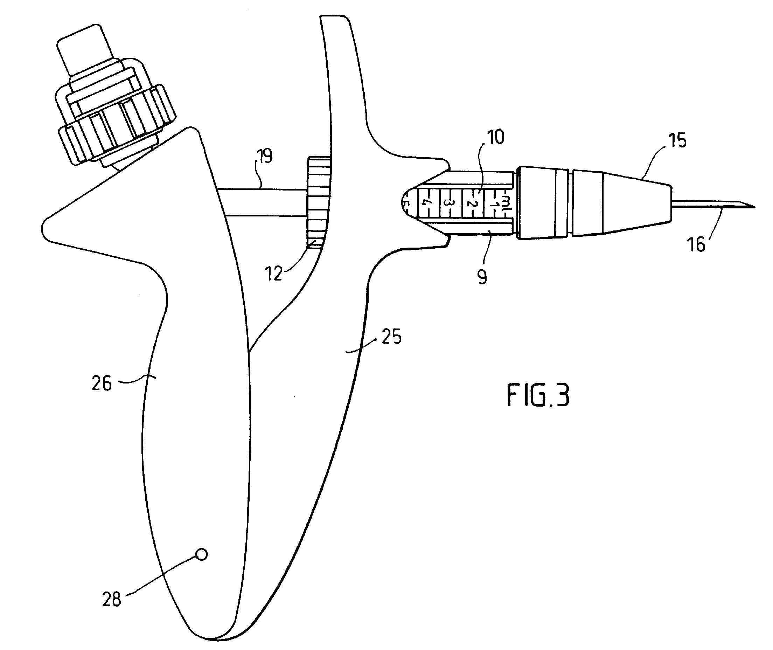

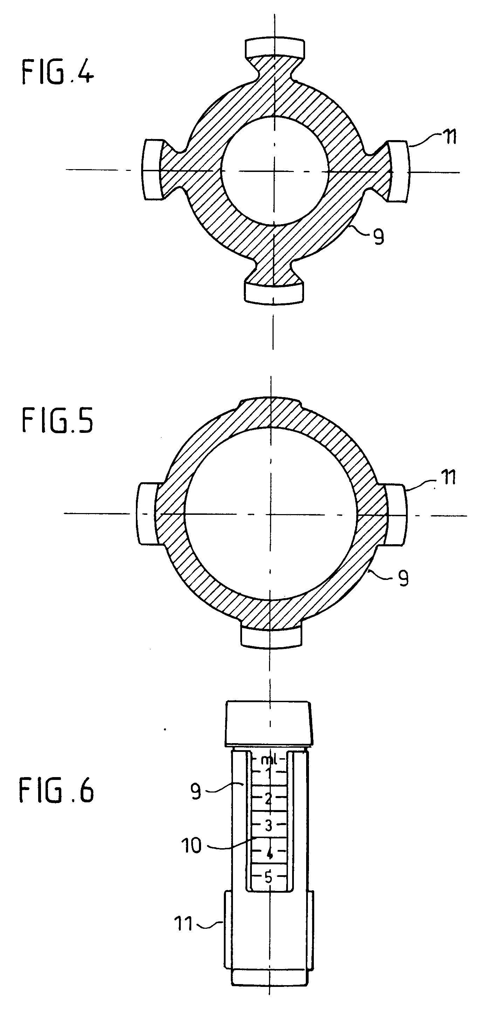

Comme le montrent les figures 3 à 6, le réservoir 9 est animé d'un mouvement axial à l'intérieur du support 27 du corps de réservoir et est susceptible d'être immobilisé dans ledit support en au moins une position appropriée correspondant à un volume d'injection choisi. Pour ce faire, le réservoir comporte sur sa surface périphérique externe un filetage 11 qui coopère avec un taraudage du support de corps de réservoir 27 de manière à pouvoir se déplacer axialement à l'intérieur dudit support 27 vers la gauche ou vers la droite dans la figure 2. Ce déplacement est réalisé manuellement, de ce fait, le corps de réservoir 9 est susceptible de se rapprocher plus ou moins de l'extrémité 20 du piston 19 disposée opposée à la tête de piston 18. Ce réservoir ou corps de seringue 9 comporte un filetage 11 qui peut être continu ou discontinu. Dans ce deuxième cas, ce filetage se présentera sous forme de bandes longitudinales 11 disposées sur la périphérie externe dudit corps de réservoir comme le montrent les figures 4 et 5. Ces bandes longitudinales filetées coopèrent avec le taraudage 30 du support 27. Le filetage du corps de réservoir 9 coopérera également avec le taraudage de l'organe d'immobilisation 12. Cette caractéristique est plus particulièrement visible à la figure 6.

Comme ce corps de réservoir est de préférence interchangeable, il est nécessaire de conserver une dimension de section externe constante, la section interne ou diamètre interne ou section de l'alésage axial du réservoir 9 pouvant quant à elle varier en fonction de la contenance du réservoir comme le montrent les figures 4 et 5, d'où l'intérêt de ces bandes 11 qui facilitent la fabrication des réservoirs. En outre, ce réservoir comporte des graduations 10. Ce réservoir est fermé par un fond 13 qui peut être monté amovible sur ledit corps du réservoir. Tel est le cas du fond représenté dans la figure 2. Ce fond est constitué par un certain nombre de pièces, une pièce 13 qui assure l'étanchéité du fond du réservoir, cette pièce 13 creuse contenant un clapet anti-retour 17 qui, en position d'injection, est comprimé contre le support du talon de l'aiguille 16 et, à l'état de repos ou d'aspiration du fluide, n'est plus en contact avec ledit support du talon de l'aiguille. La pièce 13 est vissée à l'intérieur du support du talon de l'aiguille généralement en matière plastique 14 qui assure la solidarisation de l'ensemble 13, 14 au corps 9 en venant se visser à l'intérieur du corps 9. L'ensemble est protégé par une pièce 15 qui coiffe l'ensemble en venant se fixer par vissage. Cette pièce 15 sert au maintien de l'aiguille 16. La pièce 13, qui vient directement en contact avec la tête de piston 18, est entièrement démontable du reste de l'embout 14. La tige de piston 19 et la tête de piston 18 sont formées d'une seule pièce et sont montées par vissage sur la pièce 20. Enfin, pour immobiliser le corps de réservoir dans la position requise, on utilise un contre-écrou 12 qui pénètre dans le support du corps de réservoir 27 et coopère avec un filetage 11 du corps 9 de réservoir de manière à immobiliser ce dernier à l'intérieur du support de corps de réservoir 27 en venant en butée contre un épaulement radial interne dudit support de réservoir 27, comme le montre la figure 2.

On remarquera que le support 27 du corps de réservoir constitue en réalité une collerette qui entoure le corps de réservoir 9 et ménage des oreilles de prise pour faciliter la manipulation de l'ensemble du dispositif. Le piston 19 comprend quant à lui une tige de piston à section circulaire ou non circulaire, par exemple cruciforme, ladite tige comportant à son extrémité extérieure 20 un organe de poussée et à son extrémité intérieure une tête de piston 18. Cet organe de poussée disposé à l'extrémité extérieure peut être de type connu tel qu'un poussoir ou peut être, comme le montre la figure 2, constitué par un élément de plus grande dimension que l'alésage axial du corps 9 de réservoir, cet élément comportant deux éléments latéraux en saillie constituant des axes qui pénètrent à l'intérieur de deux coulisses 22 disposées dans des gorges ou rainures appropriées 21 de la branche 26 de l'organe d'actionnement du piston. Cette configuration particulière de l'organe de poussée du piston permet de maintenir le piston dans une position parfaitement axiale par rapport à l'alésage du réservoir 9 et est due à la conception très particulière de l'organe d'actionnement du piston décrite ci-après. En effet, ces moyens d'actionnement sont constitués par une poignée à deux branches 25, 26 affectant la forme d'ailes d'oiseau et reliées par un axe de pivotement 28. Lesdites branches 25, 26 sont telles que la branche 26 est montée solidaire de l'extrémité 20 du piston 19 opposée à la tête de piston 18 et que l'autre branche 25 constitue le support 27 de corps de réservoir. Ces branches 25, 26 sont rappelées dans une position correspondant à la position limite de déplacement axial du piston vers l'extérieur, encore appelée position de recul maximum, au moyen d'un ressort à lame 24 disposé à l'intérieur desdites branches 25 et 26. Dans cette position de recul maximal, les extrémités des marches sont conformées pour venir en butée l'une contre l'autre. En raison de cette configuration particulière, on constate, lors d'une poussée dynamique sur la branche 26 pour la rapprocher de la branche 25, que l'extrémité du piston 20 opposée à celle recevant la tête de piston 18 a tendance à coulisser vers le haut à l'intérieur desdites rainures 21 de la branche 26 avant d'avoir un déplacement parfaitement linéaire.

Dans l'exemple de la figure 2, ce piston 19 est alimenté sensiblement axialement au moyen d'un tuyau 23. Dans l'exemple de la figure 3, l'alimentation est réalisée au moyen d'un flacon disposé en permanence sur, et en liaison avec, ledit piston 19.

Bien évidemment, on peut imaginer d'autres modes de réalisation de l'invention. En effet, bien que le corps de réservoir reste toujours animé d'un mouvement relatif et soit susceptible d'être déplacé axialement à l'intérieur d'un alésage du support 27 du corps de réservoir, il est possible d'imaginer que le déplacement de ce corps de réservoir et son immobilisation dans une position déterminée soient réalisés au moyen d'un organe escamotable tel qu'un bonhomme à ressort, une vis pointeau ou tout autre organe disposé sur le support du corps et qui coopère avec un ou plusieurs logements de réception dudit organe escamotable disposés sur le corps du réservoir. On peut également imaginer que la position de recul maximum du piston soit variable par exemple au moyen d'une vis disposée entre lesdites manches constitutives de la poignée au voisinage de leur point de pivotement.

Bien évidemment, la solution inverse consistant à disposer l'organe escamotable sur le corps 9 du réservoir et les logements sur le support 27 de corps du réservoir est également possible, mais plus difficile à mettre en oeuvre.

De même, on peut envisager de déplacer axialement le corps de réservoir dans le support et de l'immobiliser en une position appropriée au moyen d'un dispositif pignons/crémaillère, l'ensemble étant supporté à la fois par le support 27 du corps et le corps 9 de réservoir. De même, les organes d'actionnement du piston qui, d'une part, doivent limiter le déplacement axial vers l'extérieur de ce piston et d'autre part, ramènent ce piston à cette position initiale de recul maximal peuvent être divers. Ceux représentés à la figure 2 étaient constitués par la poignée à deux branches 25, 26 et le ressort à lame 24, les branches 25 et 26 étant montées à pivotement autour d'un axe 28.

On peut également imaginer que les moyens d'actionnement du piston soient constitués par un organe de longueur variable qui relie l'extrémité du piston 20 opposée à la tête de piston 18 au support 27 du corps de réservoir et est susceptible d'occuper une première position dite de repos dans laquelle la tête de piston 18 est à une distance déterminée D toujours identique ou réglable du support 27 de corps de réservoir et une deuxième position dite de travail dans laquelle la tête de piston 18 vient en butée contre le fond 13 du corps 9 de réservoir. Cet organe de longueur variable peut être un organe télescopique ou à coulisse dans lequel les éléments s'emboîtent les uns dans les autres, l'ensemble étant associé ou non à un moyen de rappel.

On peut également imaginer d'utiliser un actionneur à dépression linéaire. D'autres modes de réalisation de ce moyen d'actionnement sont également envisageables mais souvent plus complexes.

L'intérêt d'une telle pompe volumétrique réglable, en particulier d'une seringue automatique, réside dans la possibilité de la fabriquer entièrement par moulage en matières plastiques d'où des coûts de fabrication moindres. En outre, cette pompe peut être réalisée entièrement démontable, ce qui facilite son nettoyage.

Le fonctionnement d'un tel dispositif correspondant par exemple à la seringue de la figure 2 est le suivant. Dans un premier temps, il convient de régler le volume devant être injecté. Pour ce faire, on rend possible le déplacement axial du corps de réservoir 9 en dévissant le contre-écrou 12. On déplace ensuite le corps de réservoir 9 à la main à l'intérieur du support de corps de réservoir 27 soit en vissant, soit en dévissant selon la position initiale qui était occupée par ce réservoir et selon la dose que l'on doit injecter. Par rapport à une position prédéterminée du corps de réservoir, si l'on veut augmenter la dose à injecter, il convient de rapprocher le corps du réservoir de l'extrémité 20 du piston opposée à la tête de piston 18, c'est-à-dire de déplacer le corps de réservoir 9 vers la gauche dans la figure 2. A l'inverse, pour augmenter le volume d'injection, on déplacera le corps de réservoir 9 par rapport au support 27 vers la droite dans la figure 2. Une fois la position du corps de réservoir 9 réglée, cette position sera figée au moyen du contre-écrou 12. Lorsque l'appareil est en main, c'est-à-dire que les doigts de la main viennent reposer sur les oreilles de prise des pièces 27 et 25 et le pouce sur la branche 26, il est possible d'actionner le piston par rapprochement de la pièce 26 en direction de la pièce 25 ou l'inverse. Ce rapprochement entraîne le déplacement axial du piston à l'intérieur du corps de réservoir vers la droite dans la figure 2, entraînant l'expulsion du liquide d'injection. Lorsque la tête de piston 18 vient en butée contre le fond du corps de réservoir 9 constitué par la pièce 13, l'injection est terminée. La branche 26 revient alors à sa position initiale grâce au ressort à lame 24 entraînant de ce fait le piston dans un déplacement axial vers l'extérieur, vers la gauche dans la figure 2, et le ramenant à une distance du support de corps de piston 27 soit toujours identique, soit variable de telle sorte que la position initiale du piston soit toujours la même, soit réglable en fonction du réglage du réservoir à l'intérieur du support de corps de réservoir 27.

Pour pratiquer une nouvelle injection, on procédera de la même façon que celle décrite ci-dessus.

## Revendications

1. Pompe volumétrique réglable telle que seringue, pistolet, pipette, du type comportant un corps (9) de préférence cylindrique constituant le réservoir de la pompe et fermé par un fond (13), un piston (19) monté coulissant axialement à l'intérieur dudit corps (9) de réservoir entre une position de repos correspondant à une position de recul maximal du piston (19) lorsque ce dernier ne subit aucune force d'enfoncement axial à l'intérieur du réservoir et une position de travail dans laquelle il vient en butée contre le fond (13) du réservoir pour injecter une quantité variable de liquide contenu entre la tête (18) du piston et ledit fond (13) du corps de réservoir et un support (27) du corps de réservoir (9),
caractérisée en ce que le réservoir (9) est déplacé d'un mouvement axial à l'intérieur d'un alésage du support (27) du corps de réservoir (9) et est susceptible d'être immobilisé dans ledit support (27) en au moins une position appropriée correspondant à un volume d'injection choisi, ledit piston (19) étant fixe par rapport audit support (27) en position de repos de la pompe de sorte que la tête de piston (18) est, dans cette position de repos, à une distance déterminée du support (27) du corps de réservoir indépendamment du volume d'injection.

2. Pompe selon la revendication 1,
caractérisée en ce que le piston (19) est actionné au moyen d'un organe (25, 26, 28, 24) qui le limite dans son mouvement axial vers l'extérieur du réservoir (9) et le ramène toujours à cette position limite de manière à ce que le support (27) du corps de réservoir soit maintenu, indépendamment du volume d'injection, à une distance D déterminée toujours identique de la tête (18) du piston dans la position de repos du piston correspondant à une position de recul maximal du piston (19) lorsque ce dernier ne subit aucune force d'enfoncement axial à l'intérieur du réservoir.

3. Pompe selon la revendication 1,
caractérisée en ce que le piston (19) est actionné au moyen d'un organe qui le limite dans son mouvement axial vers l'extérieur du réservoir (9) et le ramène à une position limite de recul maximal qui peut être réglable.

4. Pompe selon l'une des revendications 1 à 3,
caractérisée en ce que le déplacement axial du corps (9) de réservoir dans le support (27) de réservoir est réalisé au moyen d'un filetage (11) disposé sur la face externe du corps (9) de réservoir qui coopère avec un taraudage (30) du support (27) du corps de réservoir.

5. Pompe selon l'une des revendication 1 à 4,
caractérisée en ce que l'organe d'immobilisation du corps de réservoir (9) dans une position appropriée à l'intérieur du support (27) de réservoir est constitué par un contre-écrou (12) vissé sur le corps (9) dudit réservoir et venant en butée au cours du vissage contre un épaulement radial interne dudit support (27) de corps de réservoir.

6. Pompe selon la revendication 1,
caractérisée en ce que le déplacement axial du corps (9) de réservoir dans le support (27) de réservoir et son immobilisation en une position appropriée est réalisé au moyen d'un organe escamotable tel qu'un bonhomme à ressort, une vis pointeau ou autre disposé sur le support (27) du corps de réservoir respectivement sur le corps (9) du réservoir et qui coopère avec un ou plusieurs logements de réception dudit organe escamotable disposés sur le corps (9) du réservoir respectivement sur le support (27) du corps de réservoir.

7. Pompe selon la revendication 1,
caractérisée en ce que le déplacement axial du corps (9) de réservoir dans le support (27) de réservoir et son immobilisation en une position appropriée est réalisé au moyen d'un dispositif pignons-crémaillère disposé de manière appropriée sur le support (27) du corps de réservoir et le corps de réservoir (9).

8. Pompe selon l'une des revendications 1 à 3,
caractérisée en ce que les moyens d'actionnement du piston sont constitués par une poignée à deux branches (25, 26) affectant la forme d'ailes d'oiseau et reliées par un axe de pivotement (28), lesdites branches (25, 26) dont l'une est montée solidaire de l'extrémité du piston (19) opposée à la tête (18) de piston et dont l'autre constitue le support (27) du corps de réservoir étant rappelées dans une position correspondant à la position limite de déplacement axial du piston vers l'extérieur dite position de recul maximum au moyen d'un organe de rappel tel qu'un ressort à lames (24) disposé à l'intérieur desdites branches (25, 26), cette position de recul maximal pouvant être réglable au moyen d'une vis de réglage des pesées entre lesdites branches (25, 26).

9. Pompe selon l'une des revendications 1 à 3,
caractérisée en ce que les moyens d'actionnement du piston sont constitués soit par un organe de longueur variable, tel qu'un organe télescopique ou à coulisse associé ou non à un moyen de rappel, soit par un actionneur linéaire à dépression, qui relie l'extrémité du piston (19) opposée à la tête (18) de piston au support (27) du corps de réservoir et est susceptible d'occuper une première position dite de repos dans laquelle la tête (18) de piston est à une distance réglable ou déterminée D toujours identique du support (27) du corps de réservoir et une deuxième position dite de travail dans laquelle la tête (18) de piston vient en butée contre le fond (13) du corps de réservoir (9).

10. Pompe selon l'une des revendications 1 à 9,
caractérisée en ce que le corps de réservoir (9) est interchangeable.

## Claims

1. Adjustable volumetric pump, such as a syringe, gun or pipette, of the type comprising a, preferably cylindrical, body (9) forming the reservoir of the pump and closed by a base (13), a piston (19) fitted such that it is axially slidable inside the said reservoir body (9) between a rest position corresponding to a maximum return position of the piston (19) when the latter is not subjected to any axial depression force inside the reservoir and a working position in which it comes into abutment with the base (13) of the reservoir so as to inject a variable quantity of liquid contained between the head (18) of the piston and the said base (13) of the reservoir body and a support (27) for the reservoir body (9),
characterised in that the reservoir (9) is displaced in an axial movement inside a bore in the support (27) of the reservoir body (9) and is capable of being immobilised in the said support (27) in at least one appropriate position corresponding to a chosen injection volume, the said piston (19) being fixed with respect to the said support (27) in the rest position of the pump in such a way that the piston head (18) is, in said rest position, a set distance away from the support (27) of the reservoir body independently of the injection volume.

2. Pump according to Claim 1,
characterised in that the piston (19) is activated by means of an element (25, 26, 28, 24) which restricts it in its axial movement towards the outside of the reservoir (9) and always brings it back to said limiting position such that the support (27) of the reservoir body is held, independently of the injection volume, at a set distance D, which is always identical, away from the head (18) of the piston in the rest position of the piston corresponding to a maximum return position of the piston (19) when the latter is not subjected to any axial depression force inside the reservoir.

3. Pump according to Claim 1,
characterised in that the piston (19) is activated by means of an element which restricts it in its axial movement towards the outside of the reservoir (9) and returns it to a limiting position of maximum return, which can be adjustable.

4. Pump according to one of Claims 1 to 3,
characterised in that the axial displacement of the reservoir body (9) in the reservoir support (27) is effected by means of a screw thread (11), arranged on the outer surface of the reservoir body (9), which interacts with an internal screw thread (30) of the support (27) of the reservoir body.

5. Pump according to one of Claims 1 to 4,
characterised in that the element for immobilisation of the reservoir body (9) in an appropriate position inside the reservoir support (27) comprises a lock-nut (12) screwed on the body (9) of the said reservoir and, during screwing, coming into abutment with an internal radial shoulder of the said reservoir body support (27).

6. Pump according to Claim 1,
characterised in that the axial displacement of the reservoir body (9) in the reservoir support (27) and its immobilisation in an appropriate position is effected by means of a retractable element such as a spring catch, a locking screw or other element arranged on the support (27) of the reservoir body or, respectively, on the body (9) of the reservoir and which interacts with one or more seats, for receiving the said retractable element, arranged on the body (9) of the reservoir or, respectively, on the support (27) of the reservoir body.

7. Pump according to Claim 1,
characterised in that the axial displacement of the reservoir body (9) in the reservoir support (27) and its immobilisation in an appropriate position is effected by means of a rack and pinion device arranged in an appropriate manner on the support (27) of the reservoir body and the reservoir body (9).

8. Pump according to one of Claims 1 to 3,
characterised in that the means for activating the piston comprise a handle with two arms (25, 26) assuming a bird wing shape and connected by a swivel pin (28), the said arms (25, 26), one of which is mounted integrally with the end of the piston (19) opposite the piston head (18) and the other of which comprises the support (27) of the reservoir body, being returned to a position corresponding to the limiting position of axial displacement of the piston towards the outside, termed the maximum return position, by means of a return element such as a leaf spring (24) arranged inside the said arms (25, 26), it being possible for said maximum return position to be adjustable by means of a screw for adjusting the force applied between the said arms (25, 26).

9. Pump according to one of Claims 1 to 3,
characterised in that the means for activating the piston comprise either an element of variable length, such as a telescopic or sliding element, which may or may not be associated with return means, or a linear activator operating by depression, which connects the end of the piston (19) opposite the piston head (18) to the support (27) of the reservoir body and is capable of taking up a first position, termed the rest position, in which the piston head (18) is at a distance, which may be adjustable or is set D and always identical, away from the support (27) of the reservoir body and a second position, termed the working position, in which the piston head (18) comes into abutment with the base (13) of the reservoir body (9).

10. Pump according to one of Claims 1 to 9,
characterised in that the reservoir body (9) is interchangeable.

## Patentansprüche

1. Volumetrisch einstellbare Pumpe, etwa eine Spritze, Pistolenspritze, Pipette, von dem Typ, der einen vorzugsweise zylindrischen Körper (9), welcher den Behälter der Pumpe bildet und durch einen Boden (13) geschlossen ist, einen Kolben (19), der axial verschieblich in dem Inneren des Körpers (9) des Behälters zwischen einer Ruhestellung, die einer maximalen Aufzugsstellung des Kolbens (19) entspricht, wenn letzterer keiner axialen Eindrückkraft in das Behälterinnere unterliegt, und einer Arbeitsstellung, in der er in Azilage gegen den Boden (13) des Behälters kommt, um eine veränderliche Menge einer zwischen dem Kopf (18) des Kolbens und dem Boden (13) des Behälterkörpers enthaltenen Flüssigkeit zu injizieren, angeordnet ist, und einen Träger (27) des Körpers des Behälters (9) aufweist,
**dadurch gekennzeichnet,** daß der Behälter (9) durch eine axiale Bewegung im Inneren einer Bohrung des Trägers (27) des Körpers des Behälters (9) verschoben wird und daß er in dem Träger (27) mindestens in einer geeigneten Position festgesetzt werden kann, die einem gewählten Injektionsvolumen entspricht, wobei sich der Kolben (19) ortsfest gegenüber dem Träger (27) in einer Ruhestellung der Pumpe derart befindet, daß sich der Kopf des Kolbens (18), in der Ruhestellung, in einem bestimmten Abstand von dem Träger (27) des Behälterkörpers unabhängig von dem Injektionsvolumen befindet.

2. Pumpe nach Anspruch 1,
**dadurch gekennzeichnet,** daß der Kolben (19) mittels eines Organes (25, 26, 28, 24) betätigt wird, das ihn in seiner axialen Bewegung aus dem Behälter (9) hinaus begrenzt und ihn stets in die Begrenzungsstellung zurückbewegt, damit der Träger (27) des Behälterkörpers, unabhängig von dem Injektionsvolumen, mit einem stets gleichen Abstand D des Kopfes (18) des Kolbens in der Kolbenruhestellung gehalten wird, welcher einer maximalen Aufzugsstellung des Kolbens (19) entspricht, wenn letzterer keiner axialen Eindrückkraft nach innen in den Behälter hinein unterworfen ist.

3. Pumpe nach Anspruch 1,
**dadurch gekennzeichnet,** daß der Kolben (19) mittels eines Organes betätigt wird, das seine axiale Bewegung aus dem Behälter (9) hinaus begrenzt und das ihn in eine maximale Aufzugsbegrenzungsstellung zurückbewegt, die einstellbar sein kann.

4. Pumpe nach einem der Ansprüche 1 bis 3,
**dadurch gekennzeichnet,** daß die axiale Verschiebung des Körpers (9) des Behälters in dem Träger (27) des Behälters mittels eines auf der Außenfläche des Körpers (9) des Behälters vorgesehenen Gewindes (11) erfolgt, das mit einem Innengewinde (30) des Trägers (27) des Behälterkörpers zusammenwirkt.

5. Pumpe nach einem der Ansprüche 1 bis 4,
**dadurch gekennzeichnet,** daß das Festsetzorgan des Körpers des Behälters (9) in einer geeigneten Stellung im Inneren des Trägers (27) des Behälters durch eine Kontermutter (12) gebildet wird, die auf dem Körper (9) des Behälters aufgeschraubt ist und im Verlauf des Schraubens gegen eine innere radiale Schulter des Trägers (27) des Behälterkörpers zur Anlage kommt.

6. Pumpe nach Anspruch 1,
**dadurch gekennzeichnet,** daß die axiale Verschiebung des Körpers (9) des Behälters in dem Träger (27) des Behälters und seine Festsetzung in einer geeigneten Stellung mittels eines einziehbaren Organs, etwa einem Federsteller, einer Stellschraube oder dergleichen, verwirklicht ist, das auf dem Träger (27) des Behälterkörpers beziehungsweise auf dem Körper (9) des Behälters angeordnet ist, und die mit einem oder mehreren Aufnahmeräumen des einziehbaren Organs zusammenwirkt, die auf dem Körper (9) des Behälters beziehungsweise auf dem Träger (27) des Behälterkörpers angeordnet sind.

7. Pumpe nach Anspruch 1,
**dadurch gekennzeichnet,** daß die axiale Verschiebung des Körpers (9) des Behälters in dem Träger (27) des Behälters und seine Festsetzung in einer geeigneten Stellung mittels einer Ritzel-Zahnstangen-Vorrichtung verwirklicht ist, die auf geeignete Weise auf dem Träger (27) des Behälterkörpers und dem Körper des Behälters (9) angeordnet ist.

8. Pumpe nach einem der Ansprüche 1 bis 3,
**dadurch gekennzeichnet,** daß die Betätigungsmittel des Kolbens aus einem Griff mit zwei Schenkeln (25, 26) bestehen, die die Form von Schwingen annehmen, und die durch eine Schwenkachse (28) miteinander verbunden sind, wobei der eine der beiden Schenkel (25, 26) fest an dem dem Kopf (18) des Kolbens gegenüberliegenden Ende des Kolbens (19) befestigt ist und wobei der Andere den Träger (27) des Behälterkörpers bildet, welche mittels eines Rückstellorgans, wie einer Blattfeder (24), die innerhalb der beiden Schenkel (25, 26) angeordnet ist, in eine Stellung zurückgestellt werden, die der Begrenzungsstellung der axialen Verschiebung des Kolbens nach außen in die maximale Aufzugsstellung entspricht, wobei die maximale Aufzugsstellung mittels einer Druckkraft-Einstellschraube zwischen den Schenkeln (25, 26) eingestellt werden kann.

9. Pumpe nach einem der Ansprüche 1 bis 3,
**dadurch gekennzeichnet,** daß die Betätigungsmittel des Kolbens gebildet sind entweder aus einem Organ veränderlicher Länge, wie einem teleskopischen oder einem zusammenschiebbaren Organ, das mit einem Rückstellmittel verbunden ist oder nicht, oder einem linearen Unterdruck-Betätigungsglied, das das dem Kopf (18) des Kolbens (19) gegenüberliegende Ende mit dem Träger (27) des Behälterkörperss verbindet, und das eine erste Stellung, nämlich die Ruhestellung, einnehmen kann, in der sich der Kopf (18) des Kolbens in einer einstellbaren oder stets gleichen Entfernung D vom Träger (27) des Behälterkörpers befindet, und einer zweiten Stellung, nämlich der Arbeitsstellung, in der der Kopf (18) des Kolbens in Anlage mit dem Boden (13) des Körpers des Behälters (9) kommt.

10. Pumpe nach einem der Ansprüche 1 bis 9,
**dadurch gekennzeichnet,** daß der Körper des Behälters (9) austauschbar ist.
